# EUROPEAN PATENT SPECIFICATION

(11) **EP 3 231 603 B1**
(45) Date of publication and mention of the grant of the patent: **10.11.2021**
(21) Application number: 15866617.2
(22) Date of filing: 10.12.2015
(51) Int. Cl.: B32B 25/08, A61F 13/49, A61F 13/511, A61F 13/514

(54) **ELASTIC LAMINATE AND ARTICLE CONTAINING SAME**
ELASTISCHES LAMINAT UND ARTIKEL DAMIT
STRATIFIÉ ÉLASTIQUE ET ARTICLE CONTENANT CELUI-CI

(30) Priority: 12.12.2014 JP 2014251362
(43) Date of publication of application: 18.10.2017
(73) Proprietor: Nitto Denko Corporation, Ibaraki-shi, Osaka 567-8680 (JP)
(72) Inventor: TAKEDA, Kohei, Ibaraki-shi Osaka 567-8680 (JP); IKISHIMA, Shinsuke, Ibaraki-shi Osaka 567-8680 (JP); UCHIDA, Shou, Ibaraki-shi Osaka 567-8680 (JP)
(74) Representative: Müller-Boré & Partner Patentanwälte PartG mbB
(86) International application number: PCT/JP2015/084622
(87) International publication number: WO 2016/093301

(56) References cited:
- EP-A1- 3 187 333
- WO-A1-2004/060665
- WO-A2-2008/038168
- JP-A- H03 128 234
- JP-A- 2003 311 884
- JP-A- 2008 001 104
- JP-A- 2009 519 844
- JP-B2- H0 694 188
- None

## Description

The present invention relates to a stretchable laminate and an article including the stretchable laminate.

Various stretchable laminates are proposed for articles such as sanitary articles, for example, diapers and masks.

As such material, a stretchable laminate including an elastomer layer and an elastomeric resin layer arranged on at least one side of the elastomer layer has been proposed (for example, Patent Literature 1).

However, such stretchable laminate of the related-art involves a problem in that its oil resistance is low. For example, the stretchable laminate involves a problem in that an aliphatic oil, such as a baby oil, makes a hole in the stretchable laminate.

In addition, when layers each formed of a resin having an elastomeric property (e.g., an olefin-based elastomer or a styrene-based elastomer) are laminated, blocking is liable to occur between the layers. Accordingly, for example, when the stretchable laminate is turned into a rolled body under a state in which the layers each formed of a resin having an elastomeric property are laminated, blocking easily occurs between the layers each formed of a resin having an elastomeric property, and hence it is liable to be difficult to rewind the laminate.

[PTL 1] US 2011/0177735 A1

WO 2008/038168 A1 describes an elastic composite having barrier properties.

WO 2004/060665 A1 describes oil-resistant elastic laminates.

EP 3 187 333 A1 describes a stretchable laminate and an article including the same.

The present invention has been made to solve the problem of the related art, and an object of the present invention is to provide a stretchable laminate that is excellent in oil resistance and is also excellent in blocking resistance. Another obj ect of the present invention is to provide an article including such stretchable laminate.

A stretchable laminate according to one embodiment of the present invention includes: a stretchable laminate, including: an elastomer layer; and an olefin-based resin layer on at least one side of the elastomer layer, in which when the stretchable laminate has been bonded and fixed onto a glass plate in a state of being extended by 100%, and 0.5 mL of a baby oil (manufactured by Pigeon Corporation, Baby Oil Q, main component: caprylic/capric triglyceride) has been dropped onto a surface of the stretchable laminate, the stretchable laminate is free of a hole 60 minutes after the dropping; the stretchable laminate is produced by co-extrusion, wherein co-extrusion is conducted by a method involving molding the laminate with a multilayer extrusion T-die molding machine or by an inflation method; the olefin-based resin layer contains a non-elastomeric olefin-based resin; and a content of the non-elastomeric olefin-based resin in the olefin-based resin layer is from 70 wt% to 100 wt%.

In a preferred embodiment, the olefin-based resin layer is arranged on each of both sides of the elastomer layer.

In a preferred embodiment, the olefin-based resin layer is directly laminated on at least one side of the elastomer layer.

In a preferred embodiment, the stretchable laminate according to the embodiment of the present invention has a thickness of from 10 µm to 500 µm.

In a preferred embodiment, a content of the non-elastomeric olefin-based resin in the olefin-based resin layer is from 95 wt% to 100 wt%.

In a preferred embodiment, the non-elastomeric olefin-based resin contains an α-olefin homopolymer.

In a preferred embodiment, the α-olefin homopolymer includes at least one kind selected from polyethylene and homopolypropylene.

In a preferred embodiment, the polyethylene includes high-density polyethylene.

In a preferred embodiment, the olefin-based resin layer has a thickness of from 2 µm to 50 µm.

In a preferred embodiment, the olefin-based resin layer has a thickness of from 2 µm to 8 µm.

In a preferred embodiment, the elastomer layer contains an olefin-based elastomer.

In a preferred embodiment, a content of the olefin-based elastomer in the elastomer layer is from 50 wt% to 100 wt%.

In a preferred embodiment, a content of the olefin-based elastomer in the elastomer layer is from 95 wt% to 100 wt%.

In a preferred embodiment, the olefin-based elastomer includes an α-olefin-based elastomer.

In a preferred embodiment, the α-olefin-based elastomer includes at least one kind selected from an ethylene-based elastomer and a propylene-based elastomer.

In a preferred embodiment, the α-olefin-based elastomer is produced by using a metallocene catalyst.

In a preferred embodiment, the elastomer layer has a thickness of from 8 µm to 450 µm.

In a preferred embodiment, the elastomer layer has a thickness of from 8 µm to 67 µm.

In a preferred embodiment, the stretchable laminate according to the embodiment of the present invention is used in a sanitary article, wherein the sanitary article is a diaper or a mask.

An article according to one embodiment of the present invention includes the stretchable laminate according to the embodiment of the present invention, wherein the article is a diaper or a mask.

According to the present invention, the stretchable laminate that is excellent in oil resistance and is also excellent in blocking resistance can be provided. The article including such stretchable laminate can also be provided.

FIG. 1 is a schematic cross-sectional view of a stretchable laminate according to a preferred embodiment of a present invention.
FIG. 2 is a S-S data graph at the time of the pre-extension of the stretchable laminate of the present invention.
FIG. 3 is a hysteresis loop graph after the pre-extension of the stretchable laminate of the present invention.

### <<<<Stretchable Laminate>>>>

A stretchable laminate of the present invention includes: an elastomer layer; and an olefin-based resin layer arranged on at least one side of the elastomer layer. That is, in the stretchable laminate of the present invention, the olefin-based resin layer may be arranged only on one side of the elastomer layer, or the olefin-based resin layer may be arranged on each of both sides of the elastomer layer. When the stretchable laminate of the present invention has such construction, the stretchable laminate of the present invention can be excellent in oil resistance, and can also be excellent in blocking resistance.

The stretchable laminate of the present invention may include any appropriate other layer to the extent that the effects of the present invention are not impaired. The number of such any appropriate other layers may be only one, or may be two or more.

In the stretchable laminate of the present invention, it is preferred that the olefin-based resin layer be directly laminated on the elastomer layer. That is, one preferred embodiment of the stretchable laminate of the present invention is an embodiment in which the olefin-based resin layer is directly laminated on at least one side of the elastomer layer. When the olefin-based resin layer is directly laminated on the elastomer layer as described above, the stretchable laminate of the present invention can be more excellent inoil resistance, and can also be more excellent inblocking resistance.

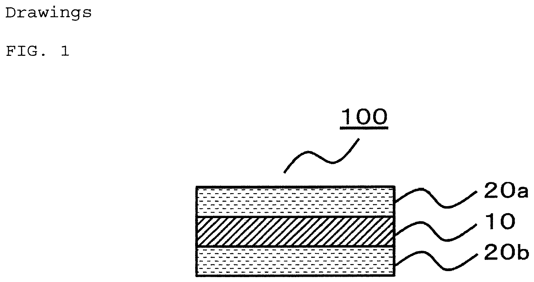

FIG. 1 is a schematic cross-sectional view of a stretchable laminate according to a preferred embodiment of the present invention. A stretchable laminate 100 illustrated in FIG. 1 includes an elastomer layer 10, an olefin-based resin layer 20a arranged on one side of the elastomer layer 10, and an olefin-based resin layer 20b arranged on the elastomer layer 10 on an opposite side to the olefin-based resin layer 20a. A material for bonding the elastomer layer 10 and the olefin-based resin layer 20a and/or for bonding the elastomer layer 10 and the olefin-based resin layer 20b may be present therebetween. Examples of such material include an adhesive, a pressure-sensitive adhesive, and a hot-melt pressure-sensitive adhesive.

The thickness of the stretchable laminate of the present invention varies depending on the thickness of the elastomer layer or the thickness of the olefin-based resin layer and is preferably from 10 µm to 500 µm, more preferably from 10 µm to 250 µm, still more preferably from 10 µm to 200 µm, particularly preferably from 10 µm to 100 µm. When the thickness of the stretchable laminate of the present invention is adjusted to fall within such range, the laminate can be easily used as a material to be used in an article such as a sanitary article, for example, a diaper or a mask.

When the stretchable laminate of the present invention is bonded and fixed onto a glass plate in a state of being extended by 100%, and 0.5 mL of a baby oil (manufactured by Pigeon Corporation, Baby Oil Q, main component: caprylic/capric triglyceride) is dropped onto the surface of the stretchable laminate, the stretchable laminate is free of a hole 60 minutes after the dropping. When the stretchable laminate of the present invention has such feature, the stretchable laminate of the present invention is excellent in oil resistance. In the present invention, the phrase "the stretchable laminate is free of a hole" in the test typically means that a visible hole or crack to be formed by the dissolution of the stretchable laminate by the baby oil is not formed. Details about a test method for such oil resistance are described later.

When the stretchable laminate of the present invention is bonded and fixed onto the glass plate in a state of being extended by 100%, and 0.5 mL of the baby oil (manufactured by Pigeon Corporation, Baby Oil Q, main component: caprylic/capric triglyceride) is dropped onto the surface of the stretchable laminate, the time period for which a state in which the stretchable laminate is free of a hole can be maintained after the dropping is preferably 2 hours, more preferably 5 hours, still more preferably 10 hours, particularly preferably 24 hours. When the time period is such preferred time period as described above, the stretchable laminate of the present invention is more excellent in oil resistance.

### <<Elastomer Layer>>

Any appropriate number may be adopted as the number of the elastomer layers . The number of such elastomer layers is preferably from 1 to 5, more preferably from 1 to 3, still more preferably 1 or 2, particularly preferably 1.

When the number of the elastomer layers is two or more, all of the respective layers may be layers of the same kind, or at least two of the layers may be layers of different kinds.

The elastomer layer may contain any appropriate resin to the extent that the effects of the present invention are not impaired. Examples of such resin include an olefin-based elastomer and a styrene-based elastomer. The elastomer layer preferably contains the olefin-based elastomer. When the elastomer layer contains the olefin-based elastomer, the stretchable laminate of the present invention can be more excellent in oil resistance.

The olefin-based elastomer may be only one kind of elastomer, or may be a blend of two or more kinds of elastomers.

When the elastomer layer contains the olefin-based elastomer, heat stability is improved and hence, for example, heat decomposition in the production of the stretchable laminate of the present invention can be suppressed. In addition, when the elastomer layer contains the olef in-based elastomer, storage stability is improved and hence the fluctuation of values for physical properties during the storage of the stretchable laminate of the present invention can be suppressed.

In addition, when the elastomer layer contains the olefin-based elastomer, steps in the production of the elastomer layer can be simplified, and hence processing cost can be suppressed. This is because of the following reason: when the olefin-based elastomer is adopted, extrusion molding can be performed by using fewer kinds of resins in the production of the elastomer layer, and hence the need for the production of a master batch can be eliminated.

The content of the olefin-based elastomer in the elastomer layer is preferably from 50 wt% to 100 wt%, more preferably from 70 wt% to 100 wt%, still more preferably from 80 wt% to 100 wt%, particularly preferably from 90 wt% to 100 wt%, most preferably from 95 wt% to 100 wt% because the effects of the present invention are expressed to a larger extent. When the content of the olefin-based elastomer in the elastomer layer is adjusted to fall within the range, the stretchable laminate of the present invention can be even more excellent in oil resistance.

Examples of the olef in-based elastomer include an olefin block copolymer, an olefin random copolymer, an ethylene copolymer, a propylene copolymer, an ethylene olefin block copolymer, a propylene olefin block copolymer, an ethylene olefin random copolymer, a propylene olefin random copolymer, an ethylene propylene random copolymer, an ethylene (1-butene) random copolymer, an ethylene (1-pentene) olefin block copolymer, an ethylene (1-hexene) random copolymer, an ethylene (1-heptene) olefin block copolymer, an ethylene (1-octene) olefin block copolymer, an ethylene (1-nonene) olefin block copolymer, an ethylene (1-decene) olefin block copolymer, a propylene ethylene olefin block copolymer, an ethylene (α-olefin) copolymer, an ethylene (α-olefin) random copolymer, an ethylene (α-olefin) block copolymer, and combinations thereof.

The olefin-based elastomer has a density of preferably from 0.890 g/cm³ to 0.830 g/cm³, more preferably from 0.888 g/cm³ to 0.835 g/cm³, still more preferably from 0.886 g/cm³ to 0.835 g/cm³, particularly preferably from 0.885 g/cm³ to 0.840 g/cm³, most preferably from 0.885 g/cm³ to 0.845 g/cm³. When the olefin-based elastomer whose density falls within the range is incorporated into the elastomer layer, the stretchable laminate of the present invention can be even more excellent in oil resistance.

The olefin-based elastomer has a MFR at 230°C and 2.16 kgf of preferably from 1.0 g/10 min to 25.0 g/10 min, more preferably from 2.0 g/10 min to 23.0 g/10 min, still more preferably from 2.0 g/10 min to 21.0 g/10 min, particularly preferably from 2.0 g/10 min to 20.0 g/10 min, most preferably from 2.0 g/10 min to 19.0 g/10 min. When the olefin-based elastomer whose MFR falls within the range is incorporated into the elastomer layer, the stretchable laminate of the present invention can be even more excellent in oil resistance.

The olefin-based elastomer is specifically preferably an α-olefin-based elastomer. That is, the α-olefin-based elastomer is a copolymer of two or more kinds of α-olefins and has elastomer characteristics. Of such α-olefin-based elastomers, any one selected from an ethylene-based elastomer, a propylene-based elastomer, and a 1-butene-based elastomer is more preferred. When such α-olefin-based elastomer is adopted as the olefin-based elastomer, the stretchable laminate of the present invention can be even more excellent in oil resistance.

Of the α-olefin-based elastomers, an ethylene-based elastomer or a propylene-based elastomer is particularly preferred. When the ethylene-based elastomer or the propylene-based elastomer is adopted as the olefin-based elastomer, the stretchable laminate of the present invention can be even more excellent in oil resistance.

The α-olef in-based elastomer is also available as a commercial product. Examples of such commercial product include some products in the "Tafmer" (trademark) series (e.g., Tafmer PN-3560) manufactured by Mitsui Chemicals, Inc., and some products in the "Vistamaxx" (trademark) series (e.g., Vistamaxx 6202 and Vistamaxx 7010) manufactured by Exxon Mobil Corporation.

The α-olefin-based elastomer is preferably produced by using a metallocene catalyst. When the α-olef in-based elastomer produced by using the metallocene catalyst is adopted, the stretchable laminate of the present invention can be even more excellent in oil resistance.

The elastomer layer may contain any appropriate other component to the extent that the effects of the present invention are not impaired. Examples of such other component include any other polymer, a tackifier, a plasticizer, an antidegradant, a pigment, a dye, an antioxidant, an antistatic agent, a lubricant, a foaming agent, a heat stabilizer, a light stabilizer, an inorganic filler, and an organic filler. Those components may be used alone or in combination thereof. The content of the other component in the elastomer layer is preferably 10 wt% or less, more preferably 7 wt% or less, still more preferably 5 wt% or less, particularly preferably 2 wt% or less, most preferably 1 wt% or less.

The thickness of the elastomer layer is preferably from 8 µm to 450 µm, more preferably from 8 µm to 220 µm, still more preferably from 8 µm to 180 µm, particularly preferably from 8 µm to 90 µm, most preferably from 8 µm to 67 µm. When the thickness of the elastomer layer is adjusted to fall within such range, a stretchable laminate having more excellent fittability can be provided.

### <<Olefin-based Resin Layer>>

Any appropriate number may be adopted as the number of the olefin-based resin layers. The number of such olefin-based resin layers is preferably from 1 to 5, more preferably from 1 to 3, still more preferably 1 or 2, particularly preferably 2 (e.g., one olefin-based resin layer is arranged on each of both sides of the elastomer layer).

When the number of the olefin-based resin layers is two or more, all of the respective layers may be layers of the same kind, or at least two of the layers may be layers of different kinds.

The olefin-based resin layer contains a non-elastomeric olefin-based resin. The non-elastomeric olefin-based resin means an olefin-based resin that is not an elastomeric olefin-based resin. When the olefin-based resin layer contains the non-elastomeric olefin-based resin, the stretchable laminate of the present invention can be more excellent in oil resistance, and can also be more excellent in blocking resistance.

The non-elastomeric olefin-based resin may be only one kind of resin, or may be a blend or copolymer of two or more kinds of resins.

The content of the non-elastomeric olefin-based resin in the olefin-based resin layer is from 70 wt% to 100 wt%, preferably from 80 wt% to 100 wt%, particularly preferably from 90 wt% to 100 wt%, most preferably from 95 wt% to 100 wt% because the effects of the present invention are expressed to a larger extent. When the content of the non-elastomeric olefin-based resin in the olefin-based resin layer is adjusted to fall within the range, the stretchable laminate of the present invention can be even more excellent in oil resistance, and can also be even more excellent in blocking resistance.

Examples of the non-elastomeric olefin-based resin include an α-olefinhomopolymer, a copolymer of two or more kinds of α-olef ins, block polypropylene, random polypropylene, and a copolymer of one or two or more kinds of α-olefins and any other vinyl monomer. A copolymerization form in any such copolymer is, for example, a block form or a random form.

Examples of the α-olefin include α-olefins each having 2 to 12 carbon atoms. Examples of such α-olefin include ethylene, propylene, 1-butene, and 4-methyl-1-pentene.

Examples of the α-olefin homopolymer include polyethylene (PE), homopolypropylene (PP), poly(1-butene), and poly(4-methyl-1-pentene) .

Examples of the polyethylene (PE) include low-density polyethylene (LDPE), linear low-density polyethylene (LLDPE), medium-density polyethylene (MDPE), and high-density polyethylene (HDPE).

The structure of the homopolypropylene (PP) may be any one of isotactic, atactic, and syndiotactic structures.

The non-elastomeric olefin-based resin preferably contains the α-olefin homopolymer, more preferably contains at least one kind selected from polyethylene (PE) and homopolypropylene (PP), and still more preferably contains at least one kind selected from high-density polyethylene (HDPE) andhomopolypropylene (PP) because the effects of the present invention can be expressed to a larger extent. When the non-elastomeric olefin-based resin contains at least one kind selected from the high-density polyethylene (HDPE) and the homopolypropylene (PP), a stretchable laminate even more excellent in oil resistance can be provided. The content of the α-olefin homopolymer in the non-elastomeric olefin-based resin is preferably from 50 wt% to 100 wt%, more preferably from 70 wt% to 100 wt%, still more preferably from 80 wt% to 100 wt%, still further more preferably from 90 wt% to 100 wt%, particularly preferably from 95 wt% to 100 wt%, most preferably substantially 100 wt% because the effects of the present invention can be expressed to a larger extent.

Examples of the copolymer of two or more kinds of α-olefins include an ethylene/propylene copolymer, an ethylene/1-butene copolymer, an ethylene/propylene/1-butene copolymer, a copolymer of ethylene/α-olefin having 5 to 12 carbon atoms, and a copolymer of propylene/α-olefin having 5 to 12 carbon atoms.

Examples of the copolymer of one or two or more kinds of α-olefins and any other vinyl monomer include an ethylene/vinyl acetate copolymer, an ethylene/acrylic acid alkyl ester copolymer, an ethylene/methacrylic acid alkyl ester copolymer, and an ethylene-non-conjugated diene copolymer.

A commercial product may be used as the non-elastomeric olefin-based resin.

The olefin-based resin layer may contain any appropriate other component to the extent that the effects of the present invention are not impaired. Examples of such other component include a releasing agent, a UV absorber, a heat stabilizer, a filler, a lubricant, a colorant (e.g., a dye), an antioxidant, an anti-eye discharge agent, an antiblocking agent, a foaming agent, and polyethyleneimine. Those components may be used alone or in combination thereof. The content of the other component in the olefin-based resin layer is preferably 10 wt% or less, more preferably 7 wt% or less, still more preferably 5 wt% or less, particularly preferably 2 wt% or less, most preferably 1 wt% or less.

Examples of the releasing agent include a fatty acid amide-based releasing agent, a silicone-based releasing agent, a fluorine-based releasing agent, and a long-chain alkyl-based releasing agent. Of those, a fatty acid amide-based releasing agent is preferred from the viewpoint that a peeling layer more excellent in balance between peelability and resistance against contamination due to bleedout can be formed, and a saturated fatty acid bisamide is more preferred. Any appropriate content may be adopted as the content of the releasing agent. Typically, the content is preferably from 0.01 wt% to 5 wt% with respect to a resin component (preferably the non-elastomeric olefin-based resin) in the olefin-based resin layer.

Examples of the UV absorber include a benzotriazole-based compound, a benzophenone-based compound, and a benzoate-based compound. Any appropriate content may be adopted as the content of the UV absorber as long as the UV absorber does not bleed out at the time of the forming. Typically, the content is preferably from 0.01 wt% to 5 wt% with respect to the resin component (preferably the non-elastomeric olefin-based resin) in the olefin-based resin layer.

Examples of the heat stabilizer include a hindered amine-based compound, a phosphorus-based compound, and a cyanoacrylate-based compound. Any appropriate content may be adopted as the content of the heat stabilizer as long as the heat stabilizer does not bleed out at the time of the forming. Typically, the content is preferably from 0.01 wt% to 5 wt% with respect to the resin component (preferably the non-elastomeric olefin-based resin) in the olefin-based resin layer.

Examples of the filler include inorganic fillers, such as talc, titanium oxide, calcium oxide, magnesium oxide, zinc oxide, titanium oxide, calcium carbonate, silica, clay, mica, barium sulfate, whisker, and magnesium hydroxide. The average particle diameter of the filler is preferably from 0.1 µm to 20 µm. Any appropriate content may be adopted as the content of the filler. Typically, the content is preferably from 1 wt% to 200 wt% with respect to the resin component (preferably the non-elastomeric olefin-based resin) in the olefin-based resin layer.

The thickness of the olefin-based resin layer is preferably from 2 µm to 50 µm, more preferably from 2 µm to 30 µm, still more preferably from 2 µm to 20 µm, particularly preferably from 2 µm to 10 µm, most preferably from 2 µm to 8 µm. When the thickness of the olefin-based resin layer is adjusted to fall within such range, the laminate can be easily used as a material to be used in an article such as a sanitary article, for example, a diaper or a mask.

### <<Production of Stretchable Laminate>>

The method of producing the stretchable laminate of the present invention is typically, for example, a production method involving molding a laminate with a multilayer extrusion T-die molding machine. For example, when a stretchable laminate formed of a laminated construction "olefin-based resin layer/elastomer layer/olefin-based resin layer" is produced, a molding material for the olefin-based resin layer, a molding material for the elastomer layer, and a molding material for the other olefin-based resin layer are co-extruded from a T-die by using a three-layer extrusion T-die molding machine to be integrated, and are then wound in a roll shape. Thus, a rolled body of the stretchable laminate can be produced. In addition to the T-die method involving using the T-die, an inflation method may also be adopted.

### <<Oil-resisting Performance of Stretchable Laminate of the Present Invention>>

As described in the foregoing, when the stretchable laminate of the present invention is bonded and fixed onto a glass plate in a state of being extended by 100%, and 0.5 mL of a baby oil (manufactured by Pigeon Corporation, Baby Oil Q, main component: caprylic/capric triglyceride) is dropped onto the surface of the stretchable laminate, the stretchable laminate is free of a hole 60 minutes after the dropping. When the stretchable laminate of the present invention has such feature, the stretchable laminate of the present invention is excellent in oil resistance. Details about a test method for such oil resistance are described later.

When the stretchable laminate of the present invention is bonded and fixed onto the glass plate in a state of being extended by 100%, and 0.5 mL of the baby oil (manufactured by Pigeon Corporation, Baby Oil Q, main component: caprylic/capric triglyceride) is dropped onto the surface of the stretchable laminate, the time period for which a state in which the stretchable laminate is free of a hole can be maintained after the dropping is preferably 2 hours, more preferably 5 hours, still more preferably 10 hours, particularly preferably 24 hours. When the time period is such preferred time period as described above, the stretchable laminate of the present invention is more excellent in oil resistance.

### <<Antiblocking Performance of Stretchable Laminate of the Present Invention>>

The stretchable laminate of the present invention includes the olefin-based resin layer on at least one side of the elastomer layer. When the stretchable laminate of the present invention includes such olefin-based resin layer, the laminate can preferably express excellent antiblocking performance. Particularly when the stretchable laminate of the present invention includes the olefin-based resin layer on each of both sides of the elastomer layer, the laminate can express extremely excellent antiblocking performance.

When a stretchable laminate free of any olefin-based resin layer on an outer layer thereof, inparticular, a stretchable laminate having elastomer layers on both of its outer layers is turned into a rolled body, blocking easily occurs between the elastomer layers, and hence it is liable to be difficult to rewind the laminate. The stretchable laminate of the present invention includes the olefin-based resin layer on at least one side of the elastomer layer. Accordingly, when the laminate is turned into a rolled body, blocking can be suppressed and hence its rewinding can be easily performed.

When the stretchable laminate of the present invention contains such filler as described in the foregoing in the olefin-based resin layer, the laminate can express more excellent antiblocking performance. However, the stretchable laminate of the present invention can express excellent antiblocking performance even when the laminate does not contain such filler as described in the foregoing in the olefin-based resin layer.

### <<Elastic Performance of Stretchable Laminate of the Present Invention>>

The stretchable laminate of the present invention can express excellent elastic performance.

The stretchable laminate of the present invention is pref erably pre-extended in order that the laminate may be caused to express excellent elastic performance. The pre-extension is pre-extension having the following meaning: the stretchable laminate of the present invention may be extended again in its final use (e.g. , at the time of the production of a diaper and at the time of the use of the diaper).

The pre-extension is preferably performed after the stretchable laminate of the present invention has been produced and sufficiently solidified.

The pre-extension may be performed on the entirety of the original length or width in at least one direction, or may be performed on part of the original length or width. In addition, the pre-extension may be performed in any appropriate direction. The pre-extension is preferably performed on the original length or width in at least one direction.

The extension degree of the pre-extension is preferably 50% or more and less than 150% (typically 100%), more preferably 150% or more and less than 250% (typically 200%), still more preferably 250% or more and less than 350% (typically 300%), particularly preferably 350% or more and less than 450% (typically 400%) . For example, 100% pre-extension means that the laminate is extended by a factor of 2. The stretchable laminate of the present invention can express more excellent elastic performance when pre-extended to such extension degree.

The pre-extension is preferably performed at a temperature less than the melting point of one of the elastomer layer and the olefin-based resin layer. The stretchable laminate of the present invention can express more excellent elastic performance when pre-extended at such temperature.

When the stretchable laminate of the present invention is preferably pre-extended as described above, the olefin-based resin layer undergoes plastic deformation or is extended beyond the brittle fracture point of the olef in-based resin layer, and hence the laminate can express excellent elastic performance . That is, it is preferred that the stretchable laminate of the present invention before the pre-extension hardly express its elasticity by virtue of the presence of the olefin-based resin layer and hence show satisfactory handleability, and meanwhile, the laminate after the pre-extension can express excellent elastic performance.

### <<Low Gloss Performance of Stretchable Laminate of the Present Invention>>

When the stretchable laminate of the present invention is preferably pre-extended, the olefin-based resin layer undergoes plastic deformation or is extended beyond the brittle fracture point of the olefin-based resin layer. The olefin-based resin layer that has undergone the plastic deformation or has been extended beyond the brittle fracture point as described above can be preferably reduced in glossiness. It is difficult to adopt a high-gloss material in an article such as a sanitary article, for example, a diaper or a mask because the material is misinterpreted as being wet. The stretchable laminate of the present invention is preferred as a material to be used in an article such as a sanitary article, for example, a diaper or a mask because the laminate can be preferably reduced in glossiness by being pre-extended.

### <<Application of Stretchable Laminate of the Present Invention>>

The stretchable laminate of the present invention can be used in any appropriate article in which the effects of the present invention can be effectively utilized. That is, the article of the present invention includes the stretchable laminate of the present invention. The article is a sanitary article and the sanitary article is a diaper (inparticular, sucha diaper that the stretchable laminate of the present invention is used as a stretchable material in an ear portion or a stretchable material in the opening portion of waist surroundings or leg surroundings (a waist band or a gather)) or a mask.

### Examples

The present invention is hereinafter specifically described by way of Examples. However, the present invention is by no means limited to these Examples. Test and evaluation methods in Examples and the like are as described below. In addition, "part (s) " means "part(s) by weight" and "%" means "wt%" unless otherwise stated.

### <Evaluation of Blocking Property>

A rolled body of a stretchable laminate or a laminate obtained in any one of Examples and Comparative Examples was cut into a size of 30 mm in its widthwise direction, and was stored in a state at 23°C×50RH% for 24 hours. After that, the resultant was rewound at a rewinding speed of 300 mm/min. The case where the stretchable laminate or the laminate ruptured at the time of the rewinding was evaluated as ×, and the case where the stretchable laminate or the laminate did not rupture at the time of the rewinding was evaluated as o.

### <Evaluation of Dropping Oil Resistance>

A stretchable laminate or a laminate obtained in any one of Examples and Comparative Examples was cut into a size measuring 50 mm in its lengthwise direction by 10 mm in its widthwise direction, and was bonded and fixed onto a glass plate in a state of being extended by 100% in the lengthwise direction. 0.5 Milliliter of a baby oil (manufactured by Pigeon Corporation, Baby Oil Q, main component: caprylic/capric triglyceride) was dropped onto the surface of the extended stretchable laminate or laminate, and an evaluation was performed on the basis of the following criteria. ×: 60 Minutes after the dropping, a hole or a crack due to dissolution by the baby oil occurs in the stretchable laminate or the laminate to rupture the stretchable laminate or the laminate. Δ: 60 Minutes after the dropping, a hole or a crack due to dissolution by the baby oil occurs in the stretchable laminate or the laminate, but the stretchable laminate or the laminate does not rupture.

○: 60 Minutes after the dropping, a hole or a crack due to dissolution by the baby oil is not formed in the stretchable laminate or the laminate.

In the present invention, a "baby oil" using caprylic/capric triglyceride as a main component, such as "Baby Oil P" available from Pigeon Corporation, can be used as the "baby oil" to be used in the dropping oil resistance test in addition to the "Baby Oil Q. "

### <Elasticity Test>

A stretchable laminate or a laminate obtained in any one of Examples and Comparative Examples was cut into a size of 30 mm in its widthwise direction, and was set in a tension testing machine (manufactured by Shimadzu Corporation: AG-20kNG) at a distance between chucks of 40 mm in its lengthwise direction. The resultant was extended by 100% at a tension speed of 300 mm/min. After having been extended by 100%, the laminate was fixed in an extended state and held at room temperature for 10 minutes. After a lapse of 10 minutes, the laminate was released from the extended state, and the initial distance between the chucks, i.e., 40 mm (A) and the length of the film after the test, i.e., (40+α) mm (B) were measured. After that, a fluctuation ratio was calculated from the expression " [{ (B) - (A) } / (A) ] ×100. " A laminate whose fluctuation ratio was more than 20% was evaluated as ×, and a laminate whose fluctuation ratio was 20% or less was evaluated as o.

### <Molding Conditions>

In each of Examples and Comparative Examples, a stretchable laminate or a laminate was molded with an extrusion T-die molding machine including three layers in three types (A layer/B layer/C layer). The molding was performed under the following extrusion temperature conditions.
A layer: 200°C
B layer: 200°C
C layer: 200°C
Die temperature: 200°C

Molding materials were subjected to co-extrusion molding from a T-die to be integrated. The resultant stretchable laminate or laminate was sufficiently solidified, and was then wound in a roll shape. Thus, a rolled body was obtained.

When pre-extension was performed, the stretchable laminate or the laminate drawn from the rolled body was pre-extended by 400% in its widthwise direction.

### <Method of measuring S-S Data>

A stretchable laminate or a laminate obtained in any one of Examples and Comparative Examples was cut into a size of 30 mm in its widthwise direction, and was set in a tension testing machine (manufactured by Shimadzu Corporation: AG-20kNG) at a distance between chucks of 40 mm in its lengthwise direction. The resultant was extended by up to 400% at a tension speed of 1, 000 mm/min, and then the extension degree was returned to 0% at a tension speed of 1,000 mm/min.

### <Method of measuring Hysteresis Data>

A stretchable laminate or a laminate obtained in any one of Examples and Comparative Examples was cut into a size of 30 mm in its widthwise direction, and was set in a tension testing machine (manufactured by Shimadzu Corporation: AG-20kNG) at a distance between chucks of 40 mm in its lengthwise direction. The resultant was extended by up to 100% at a tension speed of 500 mm/min, and then the extension degree was returned to 0% at a tension speed of 500 mm/min.

### [Example 1]

100 Parts by weight of HDPE (manufactured by Tosoh Corporation, trade name: Nipolon Hard 1000) serving as a non-elastomeric olefin-based resin was loaded into the A layer of an extruder, 100 parts by weight of an EPR (manufactured by Exxon Mobil Corporation, trade name: Vistamaxx 6202) serving as an olefin-based elastomer was loaded into the B layer of the extruder, and 100 parts by weight of HDPE (manufactured by Tosoh Corporation, trade name: Nipolon Hard 1000) serving as a non-elastomeric olefin-based resin was loaded into the C layer of the extruder, followed by the extrusion of a stretchable laminate (1) having a total thickness of 40 µm in which the thicknesses of the A layer, the B layer, and the C layer were 2 µm, 36 µm, and 2 µm, respectively.

The results of the evaluations of the stretchable laminate are shown in Table 1.

The resultant stretchable laminate (1) was pre-stretched in its widthwise direction at a ratio of 400%.

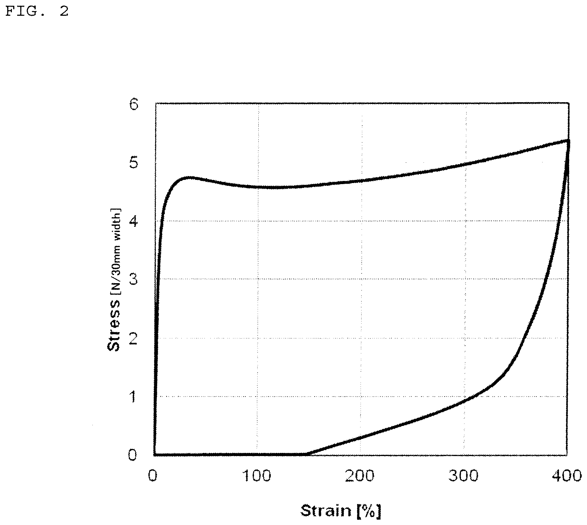

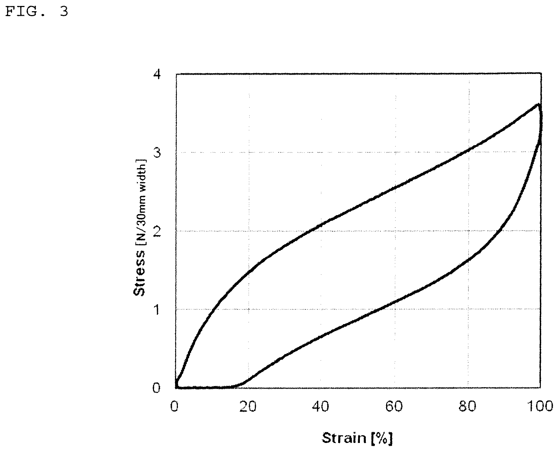

Measured S-S data at the time of the pre-extension of the stretchable laminate (1) is as shown in FIG. 2. In addition, a measured hysteresis loop after the pre-extension of the stretchable laminate (1) is as shown in FIG. 3.

### [Example 2]

A stretchable laminate (2) was obtained in the same manner as in Example 1 except that HDPE (manufactured by Basell, trade name: 52518) was used as a non-elastomeric olefin-based resin.

The results of the evaluations of the stretchable laminate are shown in Table 1.

### [Example 3]

A stretchable laminate (3) was obtained in the same manner as in Example 1 except that PP (manufactured by Japan Polypropylene Corporation, trade name: Novatec PP BC03C) was used as a non-elastomeric olefin-based resin.

The results of the evaluations of the stretchable laminate are shown in Table 1.

### [Example 4]

A stretchable laminate (4) was obtained in the same manner as in Example 1 except that a product obtained by compounding 95 parts by weight of an EPR (manufactured by Exxon Mobil Corporation, trade name: Vistamaxx 6202) serving as an olefin-based elastomer and 5 parts by weight of titanium oxide (manufactured by DuPont, trade name: Ti-Pure R103) serving as a white pigment was loaded into the B layer.

The results of the evaluations of the stretchable laminate are shown in Table 1.

### [Example 5]

A stretchable laminate (5) was obtained in the same manner as in Example 1 except that the thicknesses of the A layer, the B layer, and the C layer were set to 4 µm, 64 µm, and 4 µm, respectively, i.e., the total thickness was set to 72 µm.

The results of the evaluations of the stretchable laminate are shown in Table 1.

### [Example 6]

A stretchable laminate (6) was obtained in the same manner as in Example 1 except that the thicknesses of the A layer, the B layer, and the C layer were set to 2 µm, 20 µm, and 2 µm, respectively, i.e., the total thickness was set to 24 µm.

The results of the evaluations of the stretchable laminate are shown in Table 1.

### [Example 7]

A stretchable laminate (7) was obtained in the same manner as in Example 1 except that the thicknesses of the A layer, the B layer, and the C layer were set to 8 µm, 20 µm, and 8 µm, respectively, i.e., the total thickness was set to 36 µm.

The results of the evaluations of the stretchable laminate are shown in Table 1.

### [Example 8]

A stretchable laminate (8) was obtained in the same manner as in Example 1 except that a product obtained by compounding 80 parts by weight of HDPE (manufactured by Tosoh Corporation, trade name: Nipolon Hard 1000) and 20 parts by weight of PP (manufactured by Japan Polypropylene Corporation, trade name: Novatec PP BC03C) was used as a non-elastomeric olefin-based resin.

The results of the evaluations of the stretchable laminate are shown in Table 1.

### [Example 9]

A stretchable laminate (9) was obtained in the same manner as in Example 1 except that a product obtained by compounding 70 parts by weight of an EPR (manufactured by Exxon Mobil Corporation, trade name: Vistamaxx 6202) and 30 parts by weight of Tafmer PN-3560 (manufactured by Mitsui Chemicals, Inc.) was loaded as an olefin-based elastomer into the B layer.

The results of the evaluations of the stretchable laminate are shown in Table 1.

### [Example 10]

A stretchable laminate (10) was obtained in the same manner as in Example 1 except that 100 parts by weight of an EPR (manufactured by Exxon Mobil Corporation, trade name: Vistamaxx 7010) was used as an olefin-based elastomer.

The results of the evaluations of the stretchable laminate are shown in Table 1.

### [Example 11]

A stretchable laminate (11) was obtained in the same manner as in Example 1 except that no material was loaded into the C layer.

The results of the evaluations of the stretchable laminate are shown in Table 1.

### [Example 12]

A stretchable laminate (12) was obtained in the same manner as in Example 1 except that instead of the olefin-based elastomer, 100 parts by weight of a SIS (manufactured by Zeon Corporation, trade name: Quintac 3399) was used as a styrene-based elastomer.

The results of the evaluations of the stretchable laminate are shown in Table 1.

### [Example 13]

A stretchable laminate (13) was obtained in the same manner as in Example 1 except that instead of the olefin-based elastomer, 100 parts by weight of a SBS (manufactured by Kraton Corporation, trade name: Kraton D1191) was used as a styrene-based elastomer.

The results of the evaluations of the stretchable laminate are shown in Table 1.

### [Comparative Example 1]

A laminate (C1) was obtained in the same manner as in Example 1 except that 100 parts by weight of HDPE (manufactured by Tosoh Corporation, trade name: Nipolon Hard 1000) was used as a non-elastomeric olefin-based resin in each of all of the A layer, the B layer, and the C layer.

The results of the evaluations of the laminate are shown in Table 1.

### [Comparative Example 2]

A laminate (C2) was obtained in the same manner as in Example 1 except that 100 parts by weight of a SIS (manufactured by Zeon Corporation, trade name: Quintac 3399) was used as a styrene-based elastomer in each of all of the A layer, the B layer, and the C layer.

The results of the evaluations of the laminate are shown in Table 1.

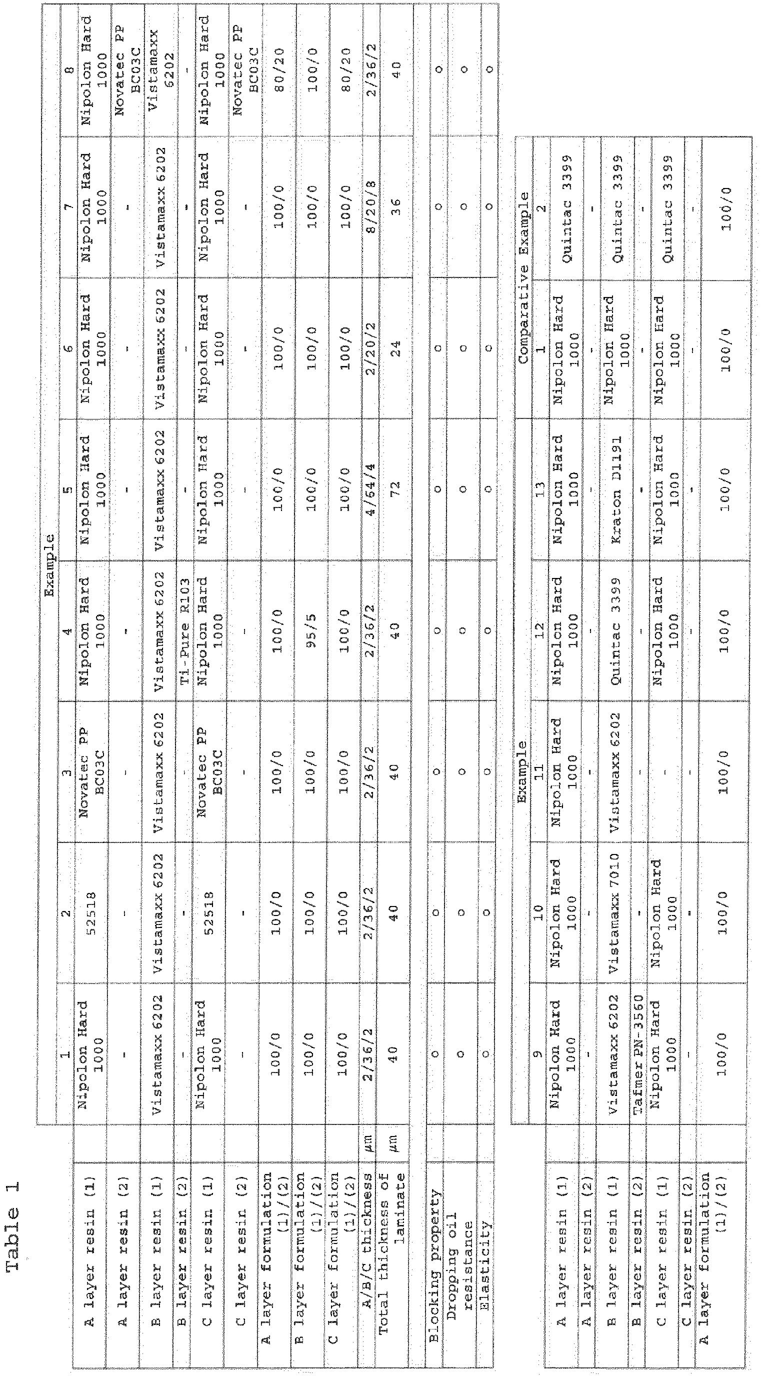

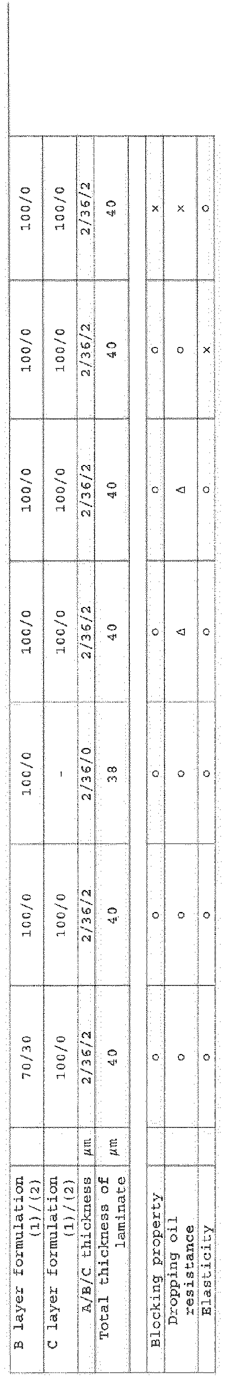

The stretchable laminate of the present invention can be used in any appropriate article in which the effects of the present invention can be effectively utilized. That is, the article of the present invention includes the stretchable laminate of the present invention. The article is a sanitary article and the sanitary article is a diaper (inparticular, such a diaper that the stretchable laminate of the present invention is used as a stretchable material in an ear portion or a stretchable material in the opening portion of waist surroundings or leg surroundings (a waist band or a gather)) or a mask.

### Reference Signs List

- 100: stretchable laminate
- 10: elastomer layer
- 20a: non-woven fabric layer
- 20b: non-woven fabric layer

## Claims

1. A stretchable laminate, comprising:
an elastomer layer; and
an olefin-based resin layer arranged on at least one side of the elastomer layer,
wherein when the stretchable laminate is bonded and fixed onto a glass plate in a state of being extended by 100%, and 0.5 mL of a baby oil as specified in the description is dropped onto a surface of the stretchable laminate, the stretchable laminate is free of a hole 60 minutes after the dropping;
the stretchable laminate is produced by co-extrusion, wherein co-extrusion is conducted by a method involving molding the laminate with a multilayer extrusion T-die molding machine or by an inflation method;
the olefin-based resin layer contains a non-elastomeric olefin-based resin; and
a content of the non-elastomeric olefin-based resin in the olefin-based resin layer is from 70 wt% to 100 wt%.

2. The stretchable laminate according to claim 1, wherein the olefin-based resin layer is arranged on each of both sides of the elastomer layer.

3. The stretchable laminate according to claim 1, wherein the olefin-based resin layer is directly laminated on at least one side of the elastomer layer.

4. The stretchable laminate according to claim 1, wherein the content of the non-elastomeric olefin-based resin in the olefin-based resin layer is from 95 wt% to 100 wt%.

5. The stretchable laminate according to claim 1, wherein the non-elastomeric olefin-based resin contains an α-olefin homopolymer.

6. The stretchable laminate according to claim 5, wherein the α-olefin homopolymer comprises at least one kind selected from polyethylene and homopolypropylene.

7. The stretchable laminate according to claim 1, wherein the olefin-based resin layer has a thickness of from 2 µm to 50 µm, preferably from 2 µm to 8 µm.

8. The stretchable laminate according to claim 1, wherein the elastomer layer contains an olefin-based elastomer.

9. The stretchable laminate according to claim 8, wherein a content of the olefin-based elastomer in the elastomer layer is from 50 wt% to 100 wt%, preferably from 95 wt% to 100 wt%.

10. The stretchable laminate according to claim 8, wherein the olefin-based elastomer comprises an α-olefin-based elastomer.

11. The stretchable laminate according to claim 10, wherein the α-olefin-based elastomer comprises at least one kind selected from an ethylene-based elastomer and a propylene-based elastomer.

12. The stretchable laminate according to claim 1, wherein the elastomer layer has a thickness of from 8 µm to 450 µm, preferably from 8 µm to 67 µm.

13. The stretchable laminate according to claim 1, wherein the stretchable laminate is used in a sanitary article, wherein the sanitary article is a diaper or a mask.

14. An article, comprising the stretchable laminate of claim 1, wherein the article is a diaper or a mask.

## Patentansprüche

1. Dehnbares Laminat, umfassend:
eine Elastomerschicht; und
eine Harzschicht auf Olefinbasis, die auf mindestens einer Seite der Elastomerschicht angeordnet ist,
wobei, wenn das dehnbare Laminat auf eine Glasplatte in einem Zustand, in dem es um 100% gedehnt ist, gebunden und fixiert ist und 0,5 mL eines Babyöls, wie in der Beschreibung angegeben, auf eine Oberfläche des dehnbaren Laminats getropft werden, das dehnbare Laminat 60 Minuten nach dem Tropfen frei von einem Loch ist;
das dehnbare Laminat durch Coextrusion hergestellt wird, wobei die Coextrusion durch ein Verfahren, das das Formen des Laminats mit einer Mehrschicht-Extrusions-T-Formwerkzeug-Formmaschine beinhaltet, oder durch ein Aufblasverfahren durchgeführt wird;
die Harzschicht auf Olefinbasis ein nicht-elastomeres Harz auf Olefinbasis enthält; und
ein Gehalt des nicht-elastomeren Harzes auf Olefinbasis in der Harzschicht auf Olefinbasis von 70 Gew.-% bis 100 Gew.-% beträgt.

2. Dehnbares Laminat nach Anspruch 1, wobei die Harzschicht auf Olefinbasis auf jeder der beiden Seiten der Elastomerschicht angeordnet ist.

3. Dehnbares Laminat nach Anspruch 1, wobei die Harzschicht auf Olefinbasis direkt auf mindestens eine Seite der Elastomerschicht laminiert ist.

4. Dehnbares Laminat nach Anspruch 1, wobei der Gehalt des nicht-elastomeren Harzes auf Olefinbasis in der Harzschicht auf Olefinbasis von 95 Gew.-% bis 100 Gew.-% beträgt.

5. Dehnbares Laminat nach Anspruch 1, wobei das nicht-elastomere Harz auf Olefinbasis ein α-Olefin-Homopolymer enthält.

6. Dehnbares Laminat nach Anspruch 5, wobei das α-Olefin-Homopolymer mindestens eine Art, ausgewählt aus Polyethylen und Homopolypropylen, umfasst.

7. Dehnbares Laminat nach Anspruch 1, wobei die Harzschicht auf Olefinbasis eine Dicke von 2 µm bis 50 µm, vorzugsweise von 2 µm bis 8 µm aufweist.

8. Dehnbares Laminat nach Anspruch 1, wobei die Elastomerschicht ein Elastomer auf Olefinbasis enthält.

9. Dehnbares Laminat nach Anspruch 8, wobei ein Gehalt des Elastomers auf Olefinbasis in der Elastomerschicht von 50 Gew.-% bis 100 Gew.-%, vorzugsweise von 95 Gew.-% bis 100 Gew.-% beträgt.

10. Dehnbares Laminat nach Anspruch 8, wobei das Elastomer auf Olefinbasis ein Elastomer auf α-Olefinbasis umfasst.

11. Dehnbares Laminat nach Anspruch 10, wobei das Elastomer auf α-Olefinbasis mindestens eine Art, ausgewählt aus einem Elastomer auf Ethylenbasis und einem Elastomer auf Propylenbasis, umfasst.

12. Dehnbares Laminat nach Anspruch 1, wobei die Elastomerschicht eine Dicke von 8 µm bis 450 µm, vorzugsweise von 8 µm bis 67 µm aufweist.

13. Dehnbares Laminat nach Anspruch 1, wobei das dehnbare Laminat in einem Sanitärartikel verwendet wird, wobei der Sanitärartikel eine Windel oder eine Maske ist.

14. Gegenstand, umfassend das dehnbare Laminat nach Anspruch 1, wobei der Gegenstand eine Windel oder eine Maske ist.

## Revendications

1. Laminé extensible, comprenant:
une couche d'élastomère; et
une couche de résine à base d'oléfine disposée sur au moins un côté de la couche d'élastomère,
dans lequel quand le laminé extensible est lié et fixé sur une plaque de verre dans un état où il est étiré de 100%, et qu'on ajoute goutte à goutte à la surface du laminé extensible 0,5 mL d'une huile de bébé, comme c'est spécifié dans la description, le laminé extensible est exempt d'un trou 60 minutes après le goutte à goutte ;
le laminé extensible est produit par co-extrusion, dans laquelle la co-extrusion est réalisée par un procédé impliquant le moulage du laminé avec une machine de moulage par extrusion à matrice en T multicouche ou par un procédé par soufflage ;
la couche de résine à base d'oléfine contient une résine à base d'oléfine non élastomère; et
la teneur en résine à base d'oléfine non élastomère dans la couche de résine à base d'oléfine, va de 70% en poids à 100% en poids.

2. Laminé extensible selon la revendication 1, dans lequel la couche de résine à base d'oléfine est disposée sur chacun des deux côtés de la couche d'élastomère.

3. Laminé extensible selon la revendication 1, dans lequel la couche de résine à base d'oléfine est directement laminée sur au moins un côté de la couche d'élastomère.

4. Stratifié extensible selon la revendication 1, dans lequel la teneur en résine à base d'oléfine non élastomère dans la couche de résine à base d'oléfine va de 95% en poids à 100% en poids.

5. Laminé extensible selon la revendication 1, dans lequel la résine à base d'oléfine non élastomère contient un homopolymère d'oléfine-α.

6. Laminé extensible selon la revendication 5, dans lequel l'homopolymère d'oléfine-α comprend au moins une sorte sélectionnée parmi le polyéthylène et l'homo-polypropylène.

7. Laminé extensible selon la revendication 1, dans lequel la couche de résine à base d'oléfine présente une épaisseur allant de 2 µm à 50 µm, de préférence de 2 µm à 8 µm.

8. Laminé extensible selon la revendication 1, dans lequel la couche d'élastomère contient un élastomère à base d'oléfine.

9. Laminé extensible selon la revendication 8, dans lequel la teneur en élastomère à base d'oléfine dans la couche d'élastomère va de 50% en poids à 100% en poids, de préférence de 95% en poids à 100% en poids.

10. Laminé extensible selon la revendication 8, dans lequel l'élastomère à base d'oléfine comprend un élastomère à base d'oléfine-a.

11. Laminé extensible selon la revendication 10, dans lequel l'élastomère à base d'oléfine-a comprend au moins une sorte sélectionnée parmi un élastomère à base d'éthylène et un élastomère à base de propylène.

12. Laminé extensible selon la revendication 1, dans lequel la couche d'élastomère présente une épaisseur allant de 8 µm à 450 µm, de préférence de 8 µm à 67 µm.

13. Laminé extensible selon la revendication 1, dans lequel le laminé extensible est utilisé dans un article sanitaire, dans lequel l'article sanitaire est un lange ou un masque.

14. Article comprenant le laminé extensible selon la revendication 1, dans lequel l'article est un lange ou un masque.
